# EUROPÄISCHE PATENTSCHRIFT

(11) **EP 0 528 164 B1**
(45) Veröffentlichungstag und Bekanntmachung des Hinweises auf die Patenterteilung: **29.05.1996**
(21) Anmeldenummer: 92111761.0
(22) Anmeldetag: 10.07.1992
(51) Int. Cl.: A61K 31/52

(54) **Verwendung von Xanthinderivaten zur Herstellung eines Arzneimittels zur Behandlung von sekundären Nervenzellschäden und Funktionsstörungen nach Schädel-Hirn-Traumen**
Use of xanthine derivatives for the manufacture of a medicament for the treatment of secondary nerve cell damages and function disorders after head-brain-traumata
Utilisation de dérivés de xanthine pour la fabrication d'un médicament pour le traitement de lésions secondaires des cellules nerveuses et de troubles de fonction après des traumatismes du crâne ou du cerveau

(30) Priorität: 11.07.1991 DE 4122884; 28.05.1992 DE 4217639
(43) Veröffentlichungstag der Anmeldung: 24.02.1993
(73) Patentinhaber: HOECHST AKTIENGESELLSCHAFT, D-65926 Frankfurt am Main (DE)
(72) Erfinder: Schubert, Hans-Peter, Prof., Dr., W-8031 Wörthsee-Steinebach (DE); Grome, John J., Dr., W-6200 Wiesbaden (DE); Kittner, Barbara, Dr., W-6200 Wiesbaden (DE); Rudolphi, Karl, Dr., W-6500 Mainz (DE); Gebert, Ulrich, Dr., W-6246 Glashütten/Tanus 2 (DE)

(56) Entgegenhaltungen:
- WO-A-85/02542
- US-A- 4 719 212
- STROKE, Bd. 19, Nr. 12, 1988, Seiten 1535-1539; JOYCE DELEO ET AL.: 'Protection against ischemic brain damage using propentofylline in gerbils'
- METABOLIC BRAIN DISEASE, Bd. 5, Nr. 1, 1990, Seiten 7-17; VLADIMIR STEFANOVICH RT AL.: 'Effect of propentofylline on the biochemical lesion of th rat brain in aluminium-induced neurotoxicity'
- DRUG DEVELOPMENT RESEARCH, Bd. 6, Nr. 4, 1985, Seiten 339-344; B.B.MRSULJA ET AL.: 'Propentofylline and postischemic brain edema: Relation to Na+-K+-ATPase activity'
- NEUROLOGY, Bd. 24, Nr. 5, 1974, Seiten 487-493; VOLKER GANSER ET AL.: 'Effect of pentoxyfylline on cerebral edema in cats'
- BIOCHEMICAL PHARMACOLOGY, Bd. 39, Nr. 11, 1990, Seiten 1813-1816; ICHIZO SHINODA ET AL.: 'Stimulation of nerve growth factor synthesis/secretion by propentofylline in cultured mouse astroglial cells'
- J. CHEM. SOC., 1945, Seiten 751-760; FREDERICK G. MANN ET AL.: 'The synthesis and properties of 1:7-dialkyl xanthines'
- ROBERT BERKOW: 'The Merck Manual', 1982, MERCK SHARP & DOHME RESEARCH LABORATORIES, RAHWAY, N.J., USA
- Head Injury, 1984
- Greenfield's Neuropathology, 4th edition, 1984
- Mechanism of Seondary Brain Damage, 1986

## Beschreibung

Eine Reihe von Oxoalkyl- und Hydroxyalkyl-xanthinen wirken durchblutungsfördernd und können auch bei cerebralen Durchblutungsstörungen eingesetzt werden (US 4,289,776, PCT 86/00401, US 3,737,433). So ist bekannt, daß 1-(5-Oxohexyl)-3-methyl-7-n-propyl-xanthin (Verbindung 1) aufgrund ihrer gefäßerweiternden Wirkung bei geringer Toxizität zur Behandlung von Patienten geeignet ist, die an arteriellen Durchblutungsstörungen leiden. Dort werden ebenfalls Verfahren zur Herstellung dieser Verbindung beschrieben (US 4,289,776).

Es ist bekannt, daß Verbindung 1 Einfluß auf die Pathophysiologie bei Gerhirnischämie hat (Stroke, Band 19, Nr. 12, 1988, Seiten 1535-1539) und bei der Behandlung von Gerhirnödemen eingesetzt wird (Drug Development Research, Band 6, 1985, Seiten 339-344; Metabolic Brain Disease, Band 5, Nr. 1, 1990, Seiten 7 - 17; Neurology, Band 24, Nr. 5, 1974, Seiten 487-493). Ferner ist bekannt, daß Verbindung 1 die Synthese von "nerve growth factor" stimuliert (Biochemical Pharmacology, Band 39, Nr. 11, 1990, Seiten 1813-1816). In der WO 85 02542 werden weitere Xanthinderivate beschrieben.

In der US 4,719,212 Patentschrift wird die Verwendung von 1-(5-Oxohexyl)-3-methyl-7-n-propyl-xanthin zur Behandlung von Gedächtnisstörungen beschrieben.

Schädel-Hirn-Traumen (SHT) spielen als Ursache für Unfalltod oder dauernde Hirnschädigung statistisch eine wesentliche Rolle. Etwa 30 % aller Verletzten bei Verkehrsunfällen erleiden SHT, die der stationären Behandlung bedürfen. Jährlich muß in der Bundesrepublik Deutschland bei jeglicher Art von Unfällen mit ca. 150.000 SHT gerechnet werden und die Zahl der Todesfälle liegt bei ca. 14.000. In den USA beträgt die Todesrate durch SHT ca. 34.000/Jahr. Viele der überlebenden SHT-Opfer erleiden langanhaltende Gesundheitsstörungen oder bleibende Behinderungen bis zur Erwerbsunfähigkeit und Dauerpflege. Die sozialmedizinischen und volkswirtschaftlichen Auswirkungen sind immens, insbesondere da die Mehrzahl der Betroffenen Verkehrsunfallopfer in relativ jungem Alter sind.

Diagnostisch werden offene und geschlossene (gedeckte) SHT unterschieden. Unter offenen werden alle Verletzungen verstanden, bei denen die Hirnhaut (Dura mater) eröffnet ist und das Gehirn über diese Öffnung mit der Außenwelt in Verbindung steht. Diese Art des SHT ist nicht Gegenstand dieser Anmeldung sondern vielmehr das geschlossene SHT. Hierbei treten focale Läsionen (z.B. Kontussionen oder Hämatome) und diffuse Hirngewebsschäden auf. Letztere breiten sich vom primär traumatisierten Bereich auf andere Hirnareale aus und können je nach Lokalisation und Schwere zu reversiblen oder bleibenden Hirnfunktionsstörungen sensorischer, motorischer oder intellektueller Art führen. Oft tritt nach SHT ein Bewußtseinsverlust auf, der in einen komatösen Zustand übergehen kann. Die primären Schäden nach Gewebszerstörung im Gehirn sind irreparabel, jedoch nur in seltenen Fällen für den tödlichen Ausgang verantwortlich. Hauptursache für bleibende Behinderung oder Tod sind vielmehr die Entstehung und das Ausmaß der sekundären Hirnschäden, die potentiell reversibel und therapeutisch beeinflußbar sind. Bei 90 % aller Patienten, die an einem SHT versterben sind Sekundärläsionen nachweisbar.

Bisher sind keine Arzneimittel bekannt, die einen wirksamen Schutz gegen die Entstehung von sekundären Hirnschäden bieten. Klinische Versuche mit Barbituraten und Calciumantagonisten blieben ohne Erfolg. Die Behandlung der Patienten mit schwerem SHT beschränkt sich daher z.Zt. auf übliche intensivmedizinische Maßnahmen wie Stabilisierung von Herz-Kreislauf und Atmung und ggf. die Kontrolle des intrazerebralen Druckes mittels Diuretika oder Osmotherapeutika. Später setzen dann physiotherapeutische und logopädische Rehabilitationsmaßnahmen ein.

Die Schwere und das Ausmaß der posttraumatischen sekundären Hirnschädigung hängen vom Ausmaß des Primärtraumas ab sowie von Art und "Timing" der ärztlichen Versorgung. Die Pathogenese der posttraumatischen Sekundärschädigung ist komplex und führt u.a. letztlich zu einem stark erhöhten intrakraniellen Druck (diffuses Hirnödem) und zur Nekrose der äußerst vulnerablen Nervenzellen (Pfenninger,E. 1988, Das Schädel-Hirn-Trauma. In: H. Bergmann. (Hsg.) Anaesthesiologie und Intensivmedizin Bd. 203, Springer-Verlag, Berlin und Head Injury: Hope through research, 1984, U.S. Dept. of Health and Human Services, National Institutes of Health Publication No. 84-2478).

Als wesentlicher pathogenetischer Faktor für Gewebsuntergang wird in der neueren Literatur die Bildung von Makrophagen diskutiert, die eine Reihe von gewebstoxischen Substanzen freisetzen, vor allem freie Sauerstoffradikale. Makrophagen werden im Zuge einer Aktivierung des Immunsystems gebildet. Sie entstehen nicht nur aus stimulierten, freie Radikale bildenden Blutzellen, sondern im Gehirn auch aus aktivierten Mikrogliazellen, die neben proteolytischen Enzymen in besonders hohem Maße freie Radikale produzieren (Banati et al.; GLia, 1991). Da eine erhöhte freie Radikalbildung offenbar zu einer Schädigung von Zellfunktionen führen kann und die neurotoxische Wirkung von Makrophagen ursächlich im Zusammenhang mit Nervenzelltod diskutiert wird können Verbindungen, die die freie Radikalbildung in Hirnmakrophagen hemmen therapeutisch in der neurologischen Klinik eingesetzt werden. Die Aktivierung von Mikrogliazellen und/oder das Auftreten von Makrophagen wird bei einer Vielzahl von neuropathologischen Prozessen, die mit dem Untergang von Hirngewebe einhergehen beobachtet (Streit et al. Glia 1, (1988), 301), so auch im Zuge der posttraumatischen sekundären Hirnschädigung.

Überraschenderweise zeigen Xanthinderivate der Formel I eine starke Inhibierung der freien Radikalbildung und zwar sowohl in peripheren (Peritoneal)-Makrophagen wie auch in Kulturen aktivierter Mikrogliazellen des Gehirns.

Die Erfindung betrifft daher die Verwendung von Xanthinderivaten der Formel I 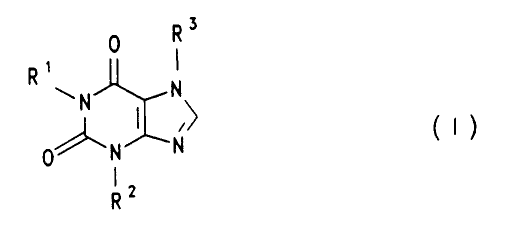
und/oder ihre physiologisch verträglichen Salze, wobei
R¹ für
a) Oxoalkyl mit 3 bis 8 Kohlenstoffatomen, deren Kohlenstoffkette geradkettig oder verzweigt sein kann,
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, oder
c) Alkyl mit 1 bis 6 Kohlenstoffatomen, deren Kohlenstoffkette geradkettig oder verzweigt sein kann, steht,
R² für
a) Wasserstoff oder
b) Alkyl mit 1 bis 4 Kohlenstoffatomen, deren Kohlenstoffkette geradkettig oder verzweigt sein kann, steht,
R³ für
a) Wasserstoff,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann,
steht, zur Herstellung von Arzneimitteln zur Behandlung von diffusen Hirngewebsschäden, die mit Mikrogliaaktivierung verbunden sind und die nach Schädel-Hirn-Traumen auftreten können.

Bevorzugt werden Xanthinderivate der Formel I verwendet, wobei
R¹ für
a) Oxoalkyl mit 4 bis 6 Kohlenstoffatomen, deren Kohlenstoffkette geradkettig ist, oder
b) Alkyl mit 3 bis 6 Kohlenstoffatomen, steht,

- R²: für Alkyl mit 1 bis 4 Kohlenstoffatomen steht,
R³ für
a) Alkyl mit 1 bis 4 Kohlenstoffatomen oder
b) Oxoalkyl mit 3 bis 6 Kohlenstoffatomen, steht.

Besonders bevorzugt wird 1-(5-Oxohexyl)-3-methyl-7-n-propyl-xanthin verwendet. Beispielhafte genannt seien die folgenden Verbindungen der Forme I:
1-(5-Hydroxy-5-methyl-hexyl)-3-methyl-xanthin,
7-(Ethoxymethyl-1-(5-hydroxy-5-methyl-hexyl)-3-methyl-xanthin,
1-(5-Oxohexyl)-3,7-dimethylxanthin,
7-(2-Oxopropyl)-1,3-di-n-butyl-xanthin oder
1-Hexyl-3,7-dimethyl-xanthin.

Geeignete physiologisch verträgliche Salze der Xanthinderivate der Formel I sind beispielsweise Alkali, Erdalkali- oder Ammoniumsalze, einschließlich solcher von physiologisch verträglichen organischen Ammoniumbasen.

Die Erfindung betrifft ferner neue Xanthinderivate der Formel I 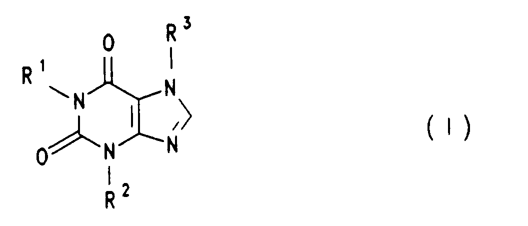
wobei
R¹ für
a) Oxoalkyl mit 3 bis 8 Kohlenstoffatomen, deren Kohlenstoffkette geradkettig oder verzweigt sein kann, oder
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, steht,

- R²: für Wasserstoff steht
R³ für
a) Wasserstoff,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann, steht.

Bevorzugt sind Xanthinderivate der Formel I, wobei
R¹ für
a) Oxoalkyl mit 4 bis 6 Kohlenstoffatomen, deren Kohlenstoffkette geradkettig ist, oder
b) Alkyl mit 3 bis 6 Kohlenstoffatomen, steht,

- R²: für Wasserstoff steht,
R³ für
a) Alkyl mit 1 bis 4 Kohlenstoffatomen oder
b) Oxoalkyl mit 3 bis 6 Kohlenstoffatomen, steht.

Die Xanthinderivate der Formel I können nach den folgenden Verfahren hergestellt werden:
a) Umsetzung von Alkalimetallsalzen von 3-Monoalkyl- oder 1,3- oder 3,7-Dialkylxanthinen mit einer Verbindung der Formel II, 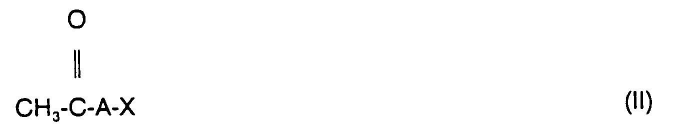 wobei A für eine Alkylgruppe mit 1 bis 6 Kohlenstoffatomen und X für Halogen, wie Fluor, Chlor, Brom oder Jod steht unter basischen Bedingungen,
b) Umsetzung eines 3-Monoalkyl- oder 3,7-Dialkylxanthins mit einer Verbindung der Formel III, 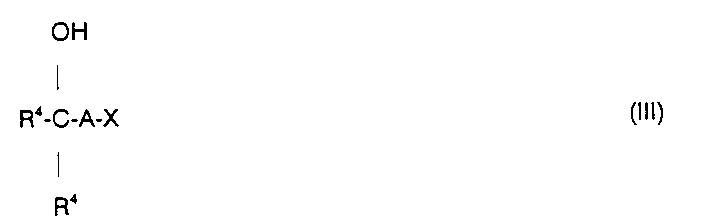 wobei X und A die unter a) genannte Bedeutung haben und R⁴ für Wasserstoff und/oder Methyl steht, unter basischen Bedingungen
c) Umsetzung von Alkalimetallsalzen von 3-Monoalkyl- oder 1,3 oder 3,7-Dialkylxanthinen mit einem entsprechenden Alkylhalogenid in einem Lösungsmittel unter basischen Bedingungen
d) Umsetzung von Alkalimetallsalzen von 3-Monoalkyl- oder 1,3-Dialkylxanthinen mit einer Verbindung der Formel IV

   CH₃-CₙH₂ₙ-O-CₘH₂ₘ-X

   worin n für eine ganze Zahl von 0 bis 4 und m für eine ganze Zahl von 1 bis 5 steht, unter der Bedingung, daß n und m zusammen nicht mehr als 5 sind, und X wie unter a) definiert ist , unter basischen Bedingungen,
e) Umsetzung von an R² und R³ geschützten Xanthinen mit einer Verbindung der Formel II oder Formel III oder einem Alkylhalogenid mit bis zu 6 C-Atomen unter basischen Bedingungen, wobei A, X und R⁴ die in b) genannte Bedeutung haben und anschließender Abspaltung der Schutzgruppe(n),
f) Umsetzung von Alkalimetallsalzen von 3-Monoalkylxanthinen oder von an R² geschützten Xanthinen mit einer Verbindung der Formel II oder Formel IV oder einem Alkylhalogenid mit bis zu 6 C-Atomen zu einem entsprechend 3,7-substituierten-Xanthin, anschließender Umsetzung mit einer Verbindung der Formel II oder Formel III oder einem Alkylhalogenid mit bis zu 6 C-Atomen und anschließender Abspaltung der gegebenenfalls vorhandenen Schutzgruppe.

Die obengenannten Umsetzungen erfolgen unter Standardbedingungen auf bekannte Weise (US 4,289,776, PCT/EP86/00401, US 3,737,433).

Unter an R² oder an R² und R³ geschützten Xanthinen werden Xanthine verstanden, die Schutzgruppen wie Benzyl, Diphenylmethyl oder 4-Methoxybenzyl in der Position von R² oder R² und R³ tragen. Die Abspaltung der Schutzgruppen erfolgt wie beispielsweise in US 4 833 146 beschrieben.

Die Ausgangsstoffe der Umsetzungen sind bekannt oder lassen sich nach literaturbekannten Methoden leicht herstellen.

Die Erfindung betrifft auch Arzneimittel, die aus mindestens einem Xanthinderivat der Formel I und/oder mindestens einem von deren physiologisch verträglichen Salze bestehen, neben pharmazeutisch geeigneten und physiologisch verträglichen Trägerstoffen, Verdünnungsmitteln und/oder andere Wirk- und Hilfsstoffe enthalten.

Die Erfindung betrifft auch ein Verfahren zur Herstellung eines erfindungsgemäßen Arzneimittels, das dadurch gekennzeichnet ist, daß man mindestens ein Xanthinderivat der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.
Die erfindungsgemäßen Arzneimittel können oral, topisch, rektal, intravenös oder gegebenenfalls auch parenteral appliziert werden. Die Applikation erfolgt nach einem erlittenen SHT.
Geeignete feste oder flüssige galenische Darreichungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglykole und Lösungsmittel, wie etwa steriles Wasser und ein- oder mehrwertige Alkohole, z.B. Glycerin, genannt.
Vorzugsweise werden die pharmazeutischen Präparate in Dosierungseinheiten hergestellt und verabreicht, wobei jede Einheit als aktiven Bestandteil eine bestimmte Dosis von mindestens einem der Xanthinderivate der Formel I und/oder mindestens einem von deren physiologisch verträglichen Salze enthält. Bei festen Dosierungseinheiten, wie Tabletten, Kapseln, Dragees oder Suppositorien, kann diese Dosis bis zu etwa 300 mg, bevorzugt jedoch etwa 10 bis 100 mg betragen. Für die Behandlung eines Patienten (70 kg), der ein SHT erlitten hat ist in frühen Phasen nach dem SHT eine intravenöse Infusionsbehandlung von maximal 1200 mg pro Tag und in der späteren Rehabilitationsphase eine orale Verabreichung von 3 mal 300 mg pro Tag der Verbindung 1 und/oder der entsprechenden Salze der Verbindung 1 indiziert.
Unter Umständen können jedoch auch höhere oder niedrigere Dosen angebracht sein. Die Verabreichung der Dosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Xanthinderivate der Formel I und/oder deren entsprechende Salze bei der Herstellung der vorgenannten galenischen Darreichungsformen auch zusammen mit anderen geeigneten Wirkstoffen, beispielsweise Wirkstoffen, die freie Sauerstoffradikale abfangen, z.B. 4H-Pyrazolo-(3,4-d)pyrimidin-4-on-1,5-dihydro oder dem Enzym Superoxiddismutase, formuliert werden.

### Beispiel 1

### Pharmakologische Prüfungen und Ergebnisse

Um die intrazelluläre Generation von freien Sauerstoff-Radikalen in Peritonealmakrophagen sowie in Kulturen aktivierter Mikroglia zu messen, wurde eine durchflußzytometrische Methode angewandt (Rothe, Oser, Valet, Naturwissenschaften, 75, 354, 1988). Speziell wurde die freie Radikalbildung in einzelnen vitalen Zellen bestimmt, indem die intrazelluläre Oxydation des membranpermeablen und nicht fluoreszierenden Dihydrorhodamin 123 (DHR; Eugene, OR, USA) zum Membran-impermeablen und intrazellulär "gefangenen", grün fluoreszierenden Rhodamin 123 gemessen wurde.

DHR wurde in einer 43.3 mM Stammlösung in N,N-Dimethylformamid (DMF; Merck, Darmstadt, F.R.G.) gelöst. Die Methode ist auch für die individuelle und simultane Messung verschiedener Unterpopulationen innerhalb einer heterogenen Zellpopulation geeignet; sie erlaubt deshalb den Ausschluß kontaminierender Populationen. Darüber hinaus wurde in einer anderen Versuchsreihe die Identifizierung des jeweils zu messenden Zelltyps durch spezifische immunzytochemische Antikörperfärbung simultan während der durchflußzytometrischen Messung sichergestellt.

Peritonealmakrophagen wurden durch Peritonealwaschung mit 10 ml HBS-Hanks (Serva Feinbiochemica, Heidelberg) von 12 Wochen alten, männlichen weißen Wistar-Ratten erhalten. Die Zellen wurden sedimentiert bei 200 g und 20° C für 5 min und resuspendiert in HBS-Hanks (4 x 106 Zellen/ml). Alle Zellen wurden nach der Präparation maximal für eine Dauer von zwei Stunden bis zur durchflußzytometrischen Analyse bei 4° C aufbewahrt.

Vor Beginn der Messung wurden alle Zellen (die Makrophagen-Suspension (10 µl) wurde weiter verdünnt mit 1 ml HBS-Hanks) für 5 min bei 37° C mit 1 µl der 43.3 mM DHR-Lösung in DMF gefärbt. Um den Effekt der Verbindung 1 zu testen, wurden in den experimentellen Gruppen die DHR-geladenen Zellen mit 10 µM oder 50 µM der erfindungsgemäßen Verbindung für 15, 25, 35, 45 und 60 min inkubiert und zwar mit oder ohne parallel laufender Stimulierung der freien Radikalbildung durch Concanavalin A (Sigma Chemie, Deisenhofen, conA, 100 µM/ml). Den jeweiligen Kontrollgruppen wurde keine Wirksubstanz zugegeben.

Mikrogliakulturen aus dem Gehirn neugeborener Ratten wurden präpariert (Giulian & Baker, J. Neuroscience, 1986, 6:2163-2178). Nach mechanischer Dissoziation des Gewebes in Dulbecco's modifiziertem Eagle's Medium (Sigma Chemie, DMEM), ergänzt mit 2 g/l NaHCO₃ und 20 % Hitze-inaktiviertem fetalen Kälberserum, wurden die Primärkulturen in 75 cm³ Kulturflaschen bei 3 % pCO₂ und 37° C für 2 bis 4 Wochen gehalten. Zellen, die auf der Oberfläche einer kontinuierenden Zellage wuchsen, wurden durch Schütteln entfernt, pelletiert und resuspendiert (3 x 106 Zellen/ml) in Hepes Hanks gepufferter Salzlösung (5 mM Hepes, 0.15 M NaCl, pH 7.35; Serva Feinbiochemica, Heidelberg, F.R.G.). Um den Effekt der Verbindung 1 zu testen, wurden in den experimentellen Gruppen die DHR-geladenen Zellen mit 50 µM der erfindungsgemäßen Verbindung für 15, 25, 35, 45 und 60 min inkubiert und zwar mit oder ohne parallel laufender Stimulierung der freien Radikalbildung durch Concanavalin A (conA, 100 µM/ml). Den jeweiligen Kontrollgruppen wurde keine Wirksubstanz zugegeben.

Das Zellvolumen und zwei Fluoreszenzen wurden simultan in etwa 10.000 Zellen pro "Sample" mit einem FACScan-Durchfluß-Zytometer (Becton Dickinson, San Jose, CA, USA) gemessen. Rhodamin 123-Grün-Fluoreszenz (500-530 nm) und Propidium-jodid-Rot-Fluoreszenz (590-700 nm) wurden gemessen bei Anregung durch einen Argon-Laser mit 488 nm Wellenlänge. Der Durchfluß-Zytometer wurde mit standardisierten gelb-grün-fluoreszierenden "Microspheres" von 4.3 µm Durchmesser (Polysciences, St. Goar, F.R.G.) kalibriert.

Jeder Meßwert basiert auf den Einzelzellmessungen der in einem "Sample" enthaltenen (ca. 10.000) Zellen. Um die experimentellen Randbedingungen möglichst konstant zu halten, wurden mehrere Experimente hintereinander am selben Tag durchgeführt. In einer solchen Versuchsreihe wurden jeweils 4 verschiedene "Samples" einer experimentellen Gruppe und ihrer Kontrolle zu verschieden definierten Zeitpunkten duchflußzytometrisch gemessen. In der Regel wurden 3-4 Versuchsreihen pro experimenteller Gruppe durchgeführt.
A) Wirkung auf Peritonealmakrophagen
Stimulation von Peritonealmakrophagen mit Concanavalin A (conA, 100 µg/ml) führte zu einem signifikanten Anstieg der Produktion freier Sauerstoffradikale, gemessen als % Zunahme der Grün-Fluoreszenz nach Oxydation von Dihydrorhodamin 123 (DHR) zu Rhodamin 123. Wenn Peritonealmakrophagen in Gegenwart von 50 µM Verbindung 1 gemessen wurden, war der stimulatorische Effekt von conA blockiert (Tab. 1). Der Effekt von Verbindung 1 ist bei Inkubationszeitpunkten über 15 min bei allen Messungen signifikant (p <0.05 im t-Test). Die gemessene % Fluoreszenz der conA-stimulierten Peritonealmakrophagen war in Gegenwart von 50 µ/M Verbindung 1 sogar geringer als in den Kontrollmessungen nicht-stimulierter Makrophagen. Der suppressive Effekt von Verbindung 1 auf die freie Radikalbildung war dosisabhängig, ein signifikanter Effekt war auch mit einer Verbindung 1-Konzentration von 10 µM Verbindung 1 zu erzielen. Hierbei wurde die % Hemmung von 10 µM Verbindung 1 auf conA-stimulierte Makrophagen bei maximaler conA-Aktivierbarkeit gemessen. Sie betrug zum Zeitpunkt 35 min: 21 % und war signifikant (p <0.05 im t-Test).

**Tabelle 1**

| Effekt von 50 µM Verbindung 1 auf die freie Radikalbildung durch conA-stimulierte Makrophagen. | | | | |
|---|---|---|---|---|
| Min | Kontrolle | conA | conA±Verb.1 | % Hemmung |
| 15 | 4.5025 (0.897) | 7.7775 (1.487) | 9.0875 (0.742) | - |
| 25 | 9.025 (2.658) | 20.2 (7.883) | 8.0867 (6.159) | 60* |
| 35 | 28.988 (2.64) | 40.47 (0.837) | 25.5 (1.87) | 37* |
| 45 | 40.015 (4.54) | 46.253 (3.12) | 31.755 (1.59) | 32* |
| Die Zahlenwerte (Mittelwerte ± S.D. in Klammern) geben die Fluoreszenzwerte als Geräte-eigene Units. * Statistisch signifikant verschieden von Kontrolle p <0.05, t-Test. | | | | |

B) Wirkung auf Mikrogliazellen
In kultivierten Mikrogliazellen war die freie Radikalbildung (gemessen als Rhodamin-Fluoreszenz) erheblich höher (ca. 50-100fach) als in Peritonealmakrophagen. Wie vorher beschrieben (Banati et al. Glia, 1991), kann diese massive freie Radikalbildung in Mikrogliazellen durch Stimulierung mit conA nicht weiter erhöht werden. Inkubation der Mikrogliazellen mit 50 µM Verbindung 1 führte zu einer deutlichen Hemmung der freien Radikalbildung. Nach einer Inkubationsdauer von 35 min in 50 µM Verbindung 1 erreichte die Depression der zellulären Rhodamin 123-Fluoreszenz ihr Maximum und betrug ca. ein Drittel der Kontrollwerte ohne Verbindung 1 (Tab. 2). Der Effekt von Verbindung 1 ist bei Inkubationszeitpunkten über 15 min bei allen Messungen signifikant (p <0.05 im t-Test).

**Tabelle 2**

| Effekt von 50 µM Verbindung 1 auf die freie Radikalbildung von kultivierten Mikrogliazellen. | | | |
|---|---|---|---|
| Min | Kontrolle | Verbindung 1 | % Hemmung |
| 15 | 2190.4 (19.5 | 2060.6 (102.3) | - |
| 25 | 2626.7 (227.4) | 2121.3 (21.4) | 19* |
| 35 | 1602.2 (125.1) | 1170.4 (27.3) | 27* |
| 45 | 2029.5 (283.5) | 1556.9 (151.9) | 23* |
| 60 | 1239.2 (108.1) | 910.4 (24.4) | 27* |
| Die Zahlenwerte (Mittelwerte ± S.D. in Klammern) geben die Fluoreszenzwerte als Geräte-eigene Units. * Statistisch signifikant verschieden von Kontrolle p <0.05, t-Test. | | | |

### Beispiel 2

### Herstellung von 1-(5-Oxohexyl)-3-methyl-7-n-propyl-xanthin (Verbindung 1)

437.2 g 3-Methyl-7-propylxanthin, in einem Gemisch aus 240 g Methanol und 321 g Wasser suspendiert, werden bei erhöhter Temperatur mit 160 g 50 %iger Natronlauge in Lösung gebracht, anschließend bei Siedetemperatur mit 358 g 1-Brom-hexanon-(5) versetzt und 4 1/2 Stunden am Rückfluß erhitzt. Nach dem Abkühlen wird nicht umgesetztes 3-Methyl-7-propylxanthin abgetrennt und der Alkohol abdestilliert. Die wässrige Lösung wird mit Natronlauge auf pH 11 gestellt und mit Methylenchlorid extrahiert. Aus dem Rückstand der Methylenchloridlösung wird nach dem Umkristallisieren aus 5.2 l Diisopropylether 1-(5-Oxohexyl)-3-methyl-7-propylxanthin vom Schmelzpunkt 69 - 70° C in etwa 90 %iger Ausbeute (bezogen auf umgesetztes 3-Methyl-7-propylxanthin) erhalten.

### Beispiel 3

### Herstellung von 7-Ethoxymethyl-1-(5-hydroxy-5-methylhexyl)-xanthin

a) 48,4 g (0,02 mol) 3-Benzylxanthin werden in einer Lösung von 8 g (0,2 mol) Natriumhydroxid in 200 ml Wasser heiß gelöst. Nach Filtration engt man im Vakuum ein, destilliert mehrmals Methanol über und trocknet das Natriumsalz im Hochvakuum.
   Das trockene Salz wird in 0,6 l Dimethylformamid (DMF) suspendiert, unter Rühren mit 18,92 g (0,2 mol)Ethoxymethylchlorid versetzt und 18 Stunden bei 110° C gerührt. Anschließend wird heiß filtriert, im Vakuum eingedampft, der Rückstand in 500 ml 2 N Natronlauge gelöst und zur Entfernung von als Nebenprodukt gebildeten 1,7- dialkylierten 3-Benzylxanthin mit Chloroform ausgeschüttet. Die alkalisch wäßrige Lösung bringt man mit 2 N Salzsäure unter Rühren auf pH 9, nutscht das gebildete Kristallisat ab, wäscht zunächst mit Wasser chloridfrei und dann mit Methanol und trocknet im Vakuum.
   Schmelzpunkt: 136 - 138° C
   C₁₅H₁₆N₄O₃ (MG= 300,3)
b) 15 g des in a) erhaltenen 7-Ethoxymethyl-3-benzylxanthins werden in 300 ml DMF mit 7,5 g (0,054 mol) Kaliumcarbonat und 8,2 g (0,054 mol) 1-Chlor-5-hydroxy -5-methylhexan (hergestellt wie in US 4 833 146) versetzt und unter Rühren 5 Stunden auf 110° C erhitzt. Man saugt heiß ab, engt ein und nimmt den Rückstand in Chloroform auf, wäscht zunächst mit 1 N Natronlauge und dann mit Wasser neutral, trocknet über Natriumsulfat, destilliert unter verminderten Druck das Lösungsmittel ab und kristallisiert den Rückstand aus Diisopropylether unter Zusatz von Essigsäureethylester um
   Ausbeute: 19,1 g (92,3% der Theorie)
   Schmelzpunkt: 96 - 97° C
   C₂₂H₃₀N₄O₄ (MG= 414,5)
c) 4,14 g (0,01 mol) des in b) erhaltenen 7-Ethoxymethyl-1-(-5-hydroxy-5-methylhexyl)-3-benzyl-xanthins werden in 100 ml Ethanol, 75 ml Wasser, 5 ml konz. NH₄OH-Lösung über 1,5 g Palladium (10%) auf Aktivkohle bei 60° C und 3,5 bar 198 Stunden unter Schütteln hydriert. Nach dem Abkühlen überlagert man mit Stickstoff, filtriert den Katalysator ab, engt ein und kristallisiert den festen Rückstand aus Essigsäureethylester um
   Ausbeute: 2,6 g (80,1 % der Theorie)
   C₁₅H₂₄N₄O₄ (MG= 324,4)

### Beispiel 4

Herstellung von 1-(5-hydroxy-5-methylhexyl)-xanthin
a) 36,3 g (0,15 mol) 3-Benzylxanthin, 3,6 g (0,15 mol) NaH in 500 ml DMF werden bei 45° C gerührt. Dann werden 25,6 g Benzylbromid gelöst in 45 ml DMF zugetropft und 5 Stunden bei 100 - 110° C erhitzt. Anschließend wird das Produkt wie in Beispiel 3a) weiter gereinigt.
b) 19,9 g (0,06 mol) des in a) erhaltenen 3,7-Dibenzylxanthins, 8,3 g Kaliumcarbonat und 10 g (0,065 mol) 1-Chlor-5-hydroxy-5-methylhexan in 350 ml DMF werden 8 Stunden unter Rühren auf 110 - 120° C erhitzt und wie unter 3b) beschrieben weiter gereinigt.
c) 4,46 (0,01 mol) des in b) erhaltenen 3,7-Dibenzyl-1-(5-hydroxy-5-methylhexyl)-xanthins werden wie in Beispiel 3c) beschrieben für 163 Stunden umgesetzt und entsprechend weiter gereinigt.
   Ausbeute: 1,53 g (57,5 % der Theorie)
   Schmelzpunkt 238 - 239° C
   C₁₂H₁₈N₄O₃ (MG= 266,3)

## Patentansprüche (Patentansprüche für folgende(n) Vertragsstaat(en): AT, BE, CH, DE, DK, FR, GB, IT, LI, LU, MC, NL, PT, SE)

1. Verwendung von mindestens einem Xanthinderivat der Formel I, 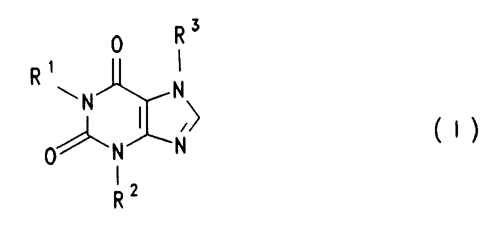 **und/oder**
mindestens einem von deren physiologisch verträglichen Salze, wobei
R¹ für
a) Oxoalkyl mit 3 bis 8 Kohlenstoffatomen, deren Kohlenstoffkette geradkettig oder verzweigt sein kann,
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, oder
c) Alkyl mit 1 bis 6 Kohlenstoffatomen, deren Kohlenstoffkette geradkettig oder verzweigt sein kann, steht,
R² für
a) Wasserstoff oder
b) Alkyl mit 1 bis 4 Kohlenstoffatomen, deren Kohlenstoffkette geradkettig oder verzweigt sein kann, steht,
R³ für
a) Wasserstoff,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann,
steht, zur Herstellung von Arzneimitteln zur Behandlung von diffusen Hirngewebsschäden, die mit Mikrogliaaktivierung verbunden sind und die nach Schädel-Hirn--Traumen auftreten können.

2. Verwendung gemäß Anspruch 1, dadurch gekennzeichnet, daß mindestens ein Xanthinderivat der Formel I, wobei R¹ für
a) Oxoalkyl mit 4 bis 6 Kohlenstoffatomen, deren Kohlenstoffkette geradkettig ist, oder
b) Alkyl mit 3 bis 6 Kohlenstoffatomen steht,
R² für Alkyl mit 1 bis 4 Kohlenstoffatomen steht,
R³ für
a) Alkyl mit 1 bis 4 Kohlenstoffatomen oder
b) Oxoalkyl mit 3 bis 6 Kohlenstoffatomen, steht und/oder mindestens einem von deren physiologisch verträglichen Salze, eingesetzt wird.

3. Verwendung von 1-(5-Oxohexyl)-3-methyl-7-n-propyl-xanthin oder mindestens einem von deren physiologisch verträglichen Salzen zur Herstellung von Arzneimitten zur Behandlung von diffusen Hirngewebsschäden, die mit Mikrogliaaktivierung verbunden sind und die nach Schädel-Hirn-Traumen auftreten können.

4. Xanthinderivat der Formel I 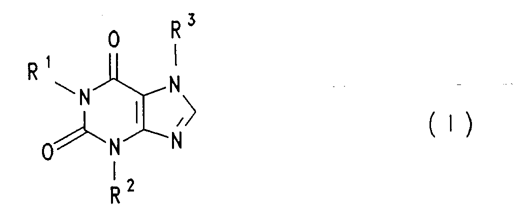 wobei
R¹ für
a) Oxoalkyl mit 3 bis 8 Kohlenstoffatomen, deren Kohlenstoffkette geradkettig oder verzweigt sein kann, oder
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt,
R² für Wasserstoff steht,
R³ für
a) Wasserstoff,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann, steht.

5. Xanthinderivat der Formel I gemäß Anspruch 4, wobei
R¹ für Hydroxyalkyl mit 1 bis 6 Kohlenstoffatomen, deren Kohlenstoffkette geradkettig, oder verzweigt ist und deren Hydroxygruppe eine primäre, sekundäre oder tertiäre Alkoholfunktion darstellt,
R² für Wasserstoff steht,
R³ für
a) Wasserstoff oder
b) Alkyl mit 1 bis 6 Kohlenstoffatomen, deren Kohlenstoffkette mit einem Sauerstoffatom unterbrochen ist, steht.

6. Verfahren zur Herstellung der Xanthinderivate der Formel I gemäß der Ansprüche 4 und 5, dadurch gekennzeichnet, daß man
a) an R² und R³ geschützte Xanthine mit einer Verbindung der Formel II oder Formel III 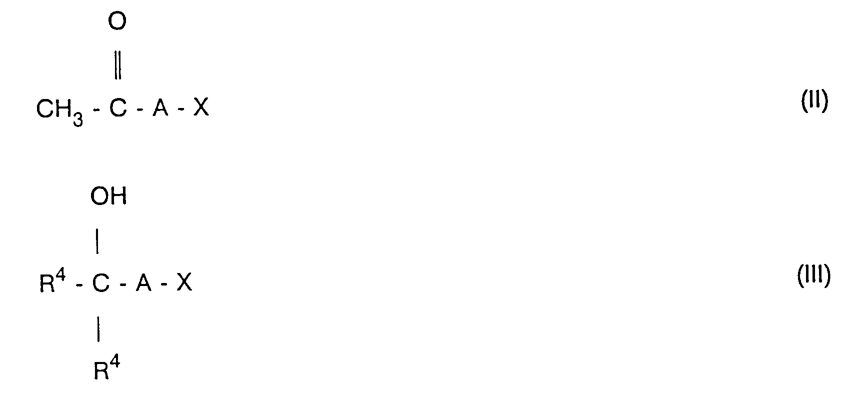 unter basischen Bedingungen umsetzt, wobei A für eine Alkylgruppe mit 1 bis 6 Kohlenstoffatomen, X für Halogen wie Fluor, Chlor, Brom oder Jod und R⁴ für Wasserstoff und/oder Methyl steht, und anschließend die Schutzgruppe abspaltet,
b) an R² geschützten Xanthinen mit einer Verbindung der Formel II oder Formel IV
CH₃ - CₙH₂ - O - CₘH₂ - X
worin n für eine ganze Zahl von 0 bis 4 und m für eine ganze Zahl von 1 bis 5 steht, unter der Bedingungen, daß n und m zusammen nicht mehr als 5 sind, und X wie unter a) definiert ist,
oder einen Alkylhalogenid mit bis zu 6 C-Atomen zu einem entsprechend 3,7-substituierten Xanthin umsetzt, anschließend mit einer Verbindung der Formel II oder Formel III umsetzt und anschließend die Schutzgruppe abspaltet.

7. Arzneimittel zur Behandlung von Erkrankungen, die nach Schädel-Hirn-Traumen auftreten können, gekennzeichnet durch einen wirksamen Gehalt von mindestens einem Xanthinderivat der Formel I gemäß der Ansprüche 4 und 5.

8. Verfahren zur Herstellung eines Arzneimittels gemäß einem oder mehreren der Ansprüche 4 und 5 zur Behandlung von Erkrankungen, die nach Schädel-Hirn-Traumen auftreten können, dadurch gekennzeichnet, daß man mindestens ein Xanthinderivat der Formel I und/oder mindestens einem von deren physiologisch verträglichen Salze mit einem physiologisch annehmbaren Träger und weiteren geeigneten Wirk-, Zusatz- oder Hilfsstoffen in eine geeignete Darreichungsform bringt.

## Patentansprüche (Patentansprüche für folgende(n) Vertragsstaat(en): ES, GR)

1. Verwendung von mindestens einem Xanthinderivat der Formel I, 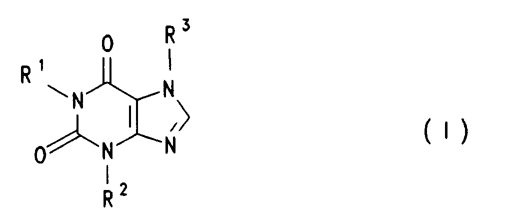 **und/oder**
mindestens einem von deren physiologisch verträglichen Salze, wobei
R¹ für
a) Oxoalkyl mit 3 bis 8 Kohlenstoffatomen, deren Kohlenstoffkette geradkettig oder verzweigt sein kann,
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, oder
c) Alkyl mit 1 bis 6 Kohlenstoffatomen, deren Kohlenstoffkette geradkettig oder verzweigt sein kann, steht,
R² für
a) Wasserstoff oder
b) Alkyl mit 1 bis 4 Kohlenstoffatomen, deren Kohlenstoffkette geradkettig oder verzweigt sein kann, steht,
R³ für
a) Wasserstoff,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann,
steht, zur Herstellung von Arzneimitteln zur Behandlung von diffusen Hirngewebsschäden, die mit Mikrogliaaktivierung verbunden sind und die nach Schädel-Hirn-Traumen auftreten können.

2. Verwendung gemäß Anspruch 1, dadurch gekennzeichnet, daß mindestens ein Xanthinderivat der Formel I, wobei
R¹ für
a) Oxoalkyl mit 4 bis 6 Kohlenstoffatomen, deren Kohlenstoffkette geradkettig ist, oder
b) Alkyl mit 3 bis 6 Kohlenstoffatomen steht,
R² für Alkyl mit 1 bis 4 Kohlenstoffatomen steht,
R³ für
a) Alkyl mit 1 bis 4 Kohlenstoffatomen oder
b) Oxoalkyl mit 3 bis 6 Kohlenstoffatomen, steht und/oder mindestens einem von deren physiologisch verträglichen Salze, eingesetzt wird.

3. Verwendung von 1-(5-Oxohexyl)-3-methyl-7-n-propyl-xanthin oder mindestens einem von deren physiologisch verträglichen Salzen zur Herstellung von Arzneimitten zur Behandlung von diffusen Hirngewebsschädigungen, die mit Mikrogliaaktivierung verbunden sind und die nach Schädel-Hirn-Traumen auftreten können.

4. Verfahren zur Herstellung der Xanthinderivate der Formel I 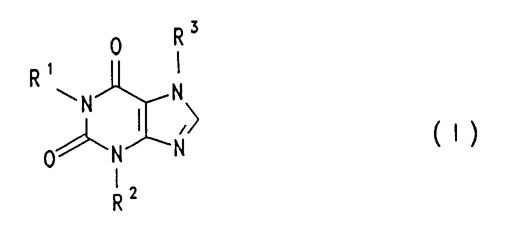 wobei
R¹ für
a) Oxoalkyl mit 3 bis 8 Kohlenstoffatomen, deren Kohlenstoffkette geradkettig oder verzweigt sein kann,
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt,
R² für Wasserstoff steht,
R³ für
a) Wasserstoff,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann,
steht, dadurch gekennzeichnet, daß man
a) an R² und R³ geschützte Xanthine mit einer Verbindung der Formel II oder Formel III 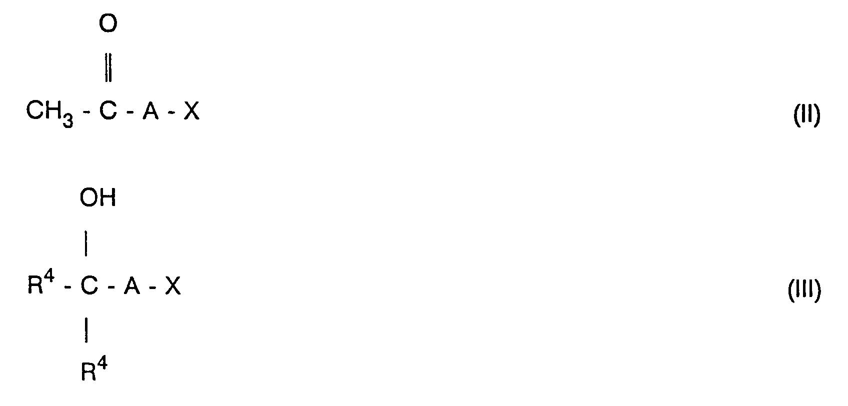 unter basischen Bedingungen umsetzt, wobei A für eine Alkylgruppe mit 1 bis 6 Kohlenstoffatomen, X für Halogen wie Fluor, Chlor, Brom oder Jod und R⁴ für Wasserstoff und/oder Methyl steht, und anschließend die Schutzgruppe abspaltet,
b) an R² geschützten Xanthinen mit einer Verbindung der Formel II oder Formel IV
CH₃ - CₙH₂ - O - CₘH₂ - X
worin n für eine ganze Zahl von 0 bis 4 und m für eine ganze Zahl von 1 bis 5 steht, unter der Bedingungen, daß n und m zusammen nicht mehr als 5 sind, und X wie unter a) definiert ist,
oder einen Alkylhalogenid mit bis zu 6 C-Atomen zu einem entsprechend 3,7-substituierten Xanthin umsetzt, anschließend mit einer Verbindung der Formel II oder Formel III umsetzt und anschließend die Schutzgruppe abspaltet.

5. Verfahren gemäß Anspruch 4, dadurch gekennzeichnet, daß man ein Xanthinderivat der Formel I herstellt, wobei
R¹ für Hydroxyalkyl mit 1 bis 8 Kohlenstoffatomen, deren Kohlenstoffkette geradkettig oder verzweigt ist und deren Hydroxygruppe eine primäre, sekundäre oder tertiäre Alkoholfunktion darstellt,
R² für Wasserstoff steht,
R³ für
a) Wasserstoff oder
b) Alkyl mit 1 bis 6 Kohlenstoffatomen, deren Kohlenstoffkette mit einem Sauerstoffatom unterbrochen ist, steht.

6. Verfahren zur Herstellung eines Arzneimittels zur Behandlung von Erkrankungen, die nach Schädel-Hirn-Traumen auftreten können, dadurch gekennzeichnet, daß man mindestens ein Xanthinderivat der Formel I gemäß der Ansprüche 4 und 5 und/oder mindestens einem von deren physiologisch verträglichen Salze mit einem physiologisch annehmbaren Träger und weiteren geeigneten Wirk-, Zusatz- oder Hilfsstoffen in eine geeignete Darreichungsform bringt.

## Claims (Claims for the following Contracting State(s): AT, BE, CH, DE, DK, FR, GB, IT, LI, LU, MC, NL, PT, SE)

1. The use of at least one xanthine derivative of the formula I 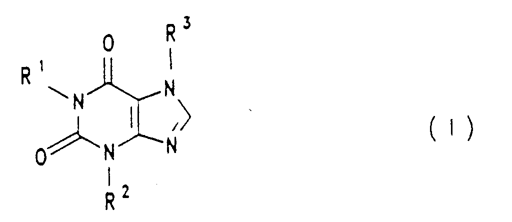 and/or at least one of its physiologically tolerable salts, in which
R¹ is
a) oxoalkyl having 3 to 8 carbon atoms, whose carbon chain can be straight-chain or branched,
b) hydroxyalkyl having 1 to 8 carbon atoms, whose carbon chain can be straight-chain or branched and whose hydroxyl group is a primary, secondary or tertiary alcohol function, or
c) alkyl having 1 to 6 carbon atoms, whose carbon chain can be straight-chain or branched,
R² is
a) hydrogen or
b) alkyl having 1 to 4 carbon atoms, whose carbon chain can be straight-chain or branched,
R³ is
a) hydrogen,
b) alkyl having 1 to 6 carbon atoms, whose carbon chain can be straight-chain or branched,
c) alkyl having 1 to 6 carbon atoms, whose carbon chain is interrupted by an oxygen atom, or
d) oxoalkyl having 3 to 8 carbon atoms, whose carbon chain can be straight-chain or branched,
for the production of pharmaceuticals for the treatment of diffuse cerebral tissue injuries which are associated with microglial activation and which can occur after cranio-cerebral traumas.

2. The use as claimed in claim 1, wherein at least one xanthine derivative of the formula I is employed in which
R¹ is
a) oxoalkyl having 4 to 6 carbon atoms, whose carbon chain is straight-chain, or
b) alkyl having 3 to 6 carbon atoms,
R² is alkyl having 1 to 4 carbon atoms,
R³ is
a) alkyl having 1 to 4 carbon atoms or
b) oxoalkyl having 3 to 6 carbon atoms, and/or at least one of its physiologically tolerable salts.

3. The use of 1-(5-oxohexyl)-3-methyl-7-n-propylxanthine or at least one of its physiologically tolerable salts for the production of pharmaceuticals for the treatment of diffuse cerebral tissue injuries which are associated with microglial activation and which can occur after cranio-cerebral traumas.

4. A xanthine derivative of the formula I 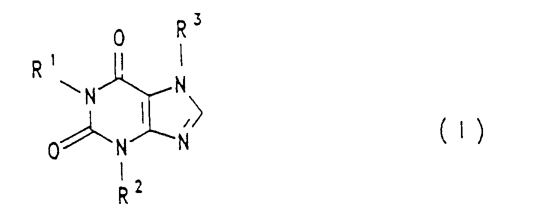 in which
R¹ is
a) oxoalkyl having 3 to 8 carbon atoms, whose carbon chain can be straight-chain or branched, or
b) hydroxyalkyl having 1 to 8 carbon atoms, whose carbon chain can be straight-chain or branched and whose hydroxyl group is a primary, secondary or tertiary alcohol function,
R² is hydrogen,
R³ is
a) hydrogen,
b) alkyl having 1 to 6 carbon atoms, whose carbon chain can be straight-chain or branched,
c) alkyl having 1 to 6 carbon atoms, whose carbon chain is interrupted by an oxygen atom, or
d) oxoalkyl having 3 to 8 carbon atoms, whose carbon chain can be straight-chain or branched.

5. A xanthine derivative of the formula I as claimed in claim 4, in which
R¹ is hydroxyalkyl having 1 to 6 carbon atoms, whose carbon chain is straight-chain or branched and whose hydroxyl group is a primary, secondary or tertiary alcohol function,
R² is hydrogen,
R³ is
a) hydrogen or
b) alkyl having 1 to 6 carbon atoms, whose carbon chain is interrupted by an oxygen atom.

6. A process for the preparation of the xanthine derivatives of the formula I as claimed in claims 4 and 5, which comprises
a) reacting xanthines protected on R² and R³ with a compound of the formula II or formula III 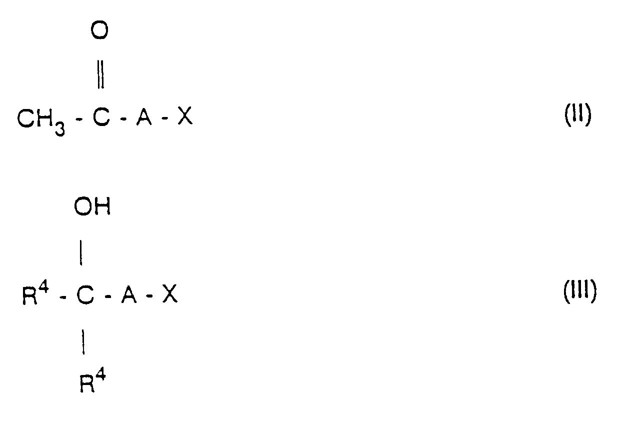 under basic conditions, where A is an alkyl group having 1 to 6 carbon atoms, X is halogen, such as fluorine, chlorine, bromine or iodine, and R⁴ is hydrogen and/or methyl, and then removing the protective group,
b) reacting xanthines protected on R² with a compound of the formula II or formula IV
CH₃-CₙH₂-O-CₘH₂-X
in which n is an integer from 0 to 4 and m is an integer from 1 to 5, with the stipulation that n and m together are not more than 5, and X is defined as in a),
or an alkyl halide having up to 6 carbon atoms to give a correspondingly 3,7-substituted xanthine, then reacting with a compound of the formula II or formula III and then removing the protective group.

7. A pharmaceutical for the treatment of disorders which can occur after cranio-cerebral traumas, which has an effective content of at least one xanthine derivative of the formula I as claimed in claims 4 and 5.

8. A process for the production of a pharmaceutical as claimed in one or more of claims 4 and 5 for the treatment of disorders which can occur after cranio-cerebral traumas, which comprises bringing at least one xanthine derivative of the formula I and/or at least one of its physiologically tolerable salts into a suitable administration form using a physiologically acceptable excipient and further suitable active substances, additives or auxiliaries.

## Claims (Claims for the following Contracting State(s): ES, GR)

1. The use of at least one xanthine derivative of the formula I 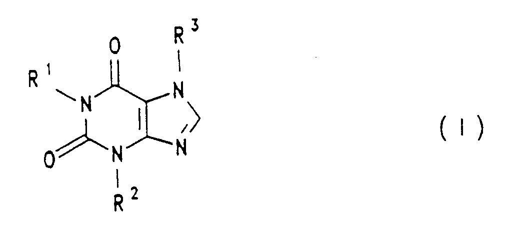 and/or at least one of its physiologically tolerable salts, in which
R¹ is
a) oxoalkyl having 3 to 8 carbon atoms, whose carbon chain can be straight-chain or branched,
b) hydroxyalkyl having 1 to 8 carbon atoms, whose carbon chain can be straight-chain or branched and whose hydroxyl group is a primary, secondary or tertiary alcohol function, or
c) alkyl having 1 to 6 carbon atoms, whose carbon chain can be straight-chain or branched,
R² is
a) hydrogen or
b) alkyl having 1 to 4 carbon atoms, whose carbon chain can be straight-chain or branched,
R³ is
a) hydrogen,
b) alkyl having 1 to 6 carbon atoms, whose carbon chain can be straight-chain or branched,
c) alkyl having 1 to 6 carbon atoms, whose carbon chain is interrupted by an oxygen atom, or
d) oxoalkyl having 3 to 8 carbon atoms, whose carbon chain can be straight-chain or branched,
for the production of pharmaceuticals for the treatment of diffuse cerebral tissue injuries which are associated with microglial activation and which can occur after cranio-cerebral traumas.

2. The use as claimed in claim 1, wherein at least one xanthine derivative of the formula I is employed in which
R¹ is
a) oxoalkyl having 4 to 6 carbon atoms, whose carbon chain is straight-chain, or
b) alkyl having 3 to 6 carbon atoms,
R² is alkyl having 1 to 4 carbon atoms,
R³ is
a) alkyl having 1 to 4 carbon atoms or
b) oxoalkyl having 3 to 6 carbon atoms, and/or at least one of its physiologically tolerable salts.

3. The use of 1-(5-oxohexyl)-3-methyl-7-n-propyl-xanthine or at least one of its physiologically tolerable salts for the production of pharmaceuticals for the treatment of diffuse cerebral tissue injuries which are associated with microglial activation and which can occur after cranio-cerebral traumas.

4. A process for the preparation of a xanthine derivative of the formula I 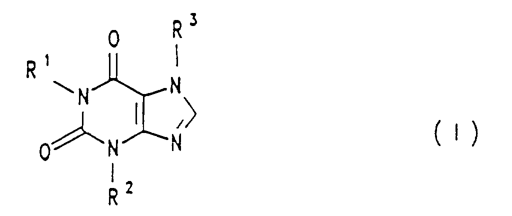 in which
R¹ is
a) oxoalkyl having 3 to 8 carbon atoms, whose carbon chain can be straight-chain or branched,
b) hydroxyalkyl having 1 to 8 carbon atoms, whose carbon chain can be straight-chain or branched and whose hydroxyl group is a primary, secondary or tertiary alcohol function,
R² is hydrogen,
R³ is
a) hydrogen,
b) alkyl having 1 to 6 carbon atoms, whose carbon chain can be straight-chain or branched,
c) alkyl having 1 to 6 carbon atoms, whose carbon chain is interrupted by an oxygen atom, or
d) oxoalkyl having 3 to 8 carbon atoms, whose carbon chain can be straight-chain or branched which comprises
a) reacting xanthines protected on R² and R³ with a compound of the formula II or formula III 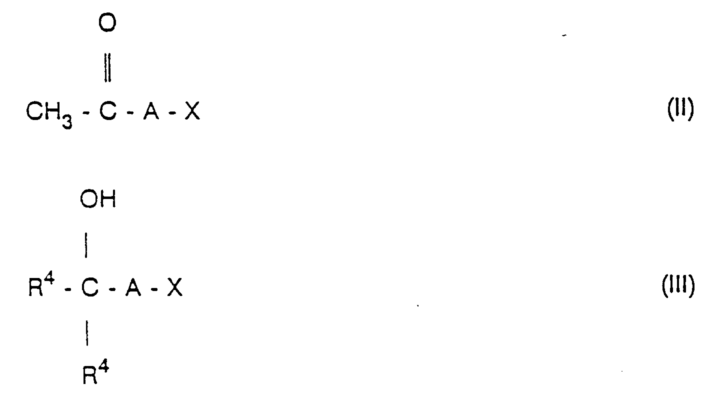 under basic conditions, where A is an alkyl group having 1 to 6 carbon atoms, X is halogen, such as fluorine, chlorine, bromine or iodine, and R⁴ is hydrogen and/or methyl, and then removing the protective group,
b) reacting xanthines protected on R² with a compound of the formula II or formula IV
CH₃-CₙH₂-O-CₘH₂-X
in which n is an integer from 0 to 4 and m is an integer from 1 to 5, with the stipulation that n and m together are not more than 5, and X is defined as in a),
or an alkyl halide having up to 6 carbon atoms to give a correspondingly 3,7-substituted xanthine, then reacting with a compound of the formula II or formula III and then removing the protective group.

5. The process as claimed in claim 4, wherein a xanthine derivative of the formula I is prepared in which
R¹ is hydroxyalkyl having 1 to 8 carbon atoms, whose carbon chain is straight-chain or branched and whose hydroxyl group is a primary, secondary or tertiary alcohol function,
R² is hydrogen,
R³ is
a) hydrogen or
b) alkyl having 1 to 6 carbon atoms, whose carbon chain is interrupted by an oxygen atom.

6. A process for the production of a pharmaceutical for the treatment of disorders which can occur after cranio-cerebral traumas, which comprises bringing at least one xanthine derivative of the formula I as claimed in claims 4 and 5 and/or at least one of its physiologically tolerable salts into a suitable administration form using a physiologically acceptable excipient and further suitable active substances, additives or auxiliaries.

## Revendications (Revendications pour l'(les) Etat(s) contractant(s) suivant(s): AT, BE, CH, DE, DK, FR, GB, IT, LI, LU, MC, NL, PT, SE)

1. Utilisation d'au moins un dérivé de xanthine de formule I, 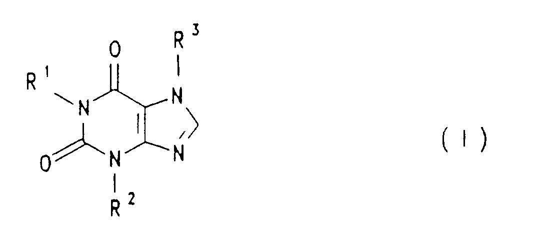 et/ou
d'au moins un de ses sels physiologiquement acceptables, formule dans laquelle
R¹ représente
a) un groupe oxoalkyle ayant de 3 à 8 atomes de carbone, dont la chaîne carbonée peut être linéaire ou ramifiée,
b) un groupe hydroxyalkyle ayant de 1 à 8 atomes de carbone, dont la chaîne carbonée peut être linéaire ou ramifiée et dont le radical hydroxy représente une fonction alcool primaire, secondaire ou tertiaire, ou
c) un groupe alkyle ayant de 1 à 6 atomes de carbone, dont la chaîne carbonée peut être linéaire ou ramifiée,
R² représente
a) un atome d'hydrogène ou
b) un groupe alkyle ayant de 1 à 4 atomes de carbone, dont la chaîne carbonée peut être linéaire ou ramifiée,
R³ représente
a) un atome d'hydrogène,
b) un groupe alkyle ayant de 1 à 6 atomes de carbone, dont la chaîne carbonée peut être linéaire ou ramifiée,
c) un groupe alkyle ayant de 1 à 6 atomes de carbone, dont la chaîne carbonée est interrompue par un atome d'oxygène, ou
d) un groupe oxoalkyle ayant de 3 à 8 atomes de carbone, dont la chaîne carbonée peut être linéaire ou ramifiée,
pour la fabrication de médicaments destinés au traitement de lésions diffuses du tissu cérébral qui sont en relation avec l'activation de la microglie et qui peuvent apparaître après des traumatismes crânio-cérébraux.

2. Utilisation selon la revendication 1, caractérisée en ce que l'on utilise au moins un dérivé de xanthine de formule I dans lequel
R¹ représente
a) un groupe oxoalkyle ayant de 4 à 6 atomes de carbone, dont la chaîne carbonée est linéaire, ou
b) un groupe alkyle ayant de 3 à 6 atomes de carbone,
R² représente un groupe alkyle ayant de 1 à 4 atomes de carbone,
R³ représente
a) un groupe alkyle ayant de 1 à 4 atomes de carbone, ou
b) un groupe oxoalkyle ayant de 3 à 6 atomes de carbone, et/ou au moins un de ses sels physiologiquement acceptables.

3. Utilisation de la 1-(5-oxohexyl)-3-méthyl-7-n-propylxanthine ou d'au moins un de ses sels physiologiquement acceptables, pour la fabrication de médicaments destinés au traitement de lésions diffuses du tissu cérébral qui sont en relation avec l'activation de la microglie et qui peuvent apparaître après des traumatismes crânio-cérébraux.

4. Dérivé de xanthine de formule I 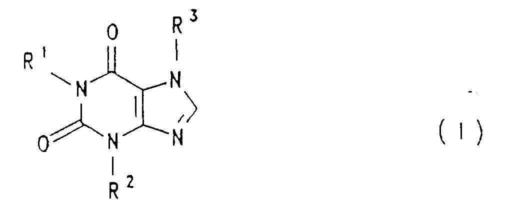 dans laquelle
R¹ représente
a) un groupe oxoalkyle ayant de 3 à 8 atomes de carbone, dont la chaîne carbonée peut être linéaire ou ramifiée, ou
b) un groupe hydroxyalkyle ayant de 1 à 8 atomes de carbone, dont la chaîne carbonée peut être linéaire ou ramifiée et dont le radical hydroxy représente une fonction alcool primaire, secondaire ou tertiaire,
R² représente un atome d'hydrogène,
R³ représente
a) un atome d'hydrogène,
b) un groupe alkyle ayant de 1 à 6 atomes de carbone, dont la chaîne carbonée peut être linéaire ou ramifiée,
c) un groupe alkyle ayant de 1 à 6 atomes de carbone, dont la chaîne carbonée est interrompue par un atome d'oxygène, ou
d) un groupe oxoalkyle ayant de 3 à 8 atomes de carbone, dont la chaîne carbonée peut être linéaire ou ramifiée.

5. Dérivé de xanthine de formule I selon la revendication 4, dans lequel
R¹ représente un groupe hydroxyalkyle ayant de 1 à 6 atomes de carbone, dont la chaîne carbonée est linéaire ou ramifiée, et dont le radical hydroxy représente une fonction alcool primaire, secondaire ou tertiaire,
R² représente un atome d'hydrogène,
R³ représente
a) un atome d'hydrogène ou
b) un groupe alkyle ayant de 1 à 6 atomes de carbone, dont la chaîne carbonée est interrompue par un atome d'oxygène.

6. Procédé pour la préparation des dérivés de xanthine de formule I selon les revendications 4 et 5, caractérisé en ce que
a) on fait réagir des xanthines protégées sur R² et R³, avec un composé de formule II ou de formule III 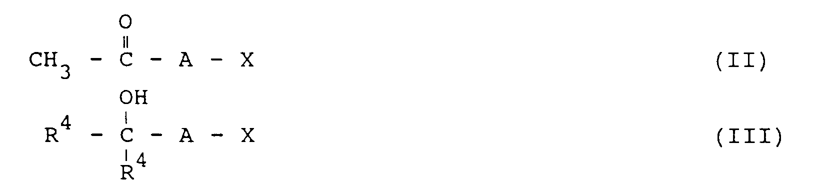 dans des conditions basiques, A représentant un groupe alkyle ayant de 1 à 6 atomes de carbone, X représentant un atome d'halogène, tel qu'un atome de fluor, chlore, brome ou iode, et R⁴ représentant un atome d'hydrogène et/ou le groupe méthyle, et ensuite on élimine le groupe protecteur,
b) on fait réagir des xanthines protégées sur R², avec un composé de formule II ou de formule IV
CH₃-CₙH₂-O-CₘH₂-X
dans laquelle
n représente un nombre entier allant de 0 à 4 et m représente un nombre entier allant de 1 à 5, étant entendu que la somme de n et m n'excède pas 5 et X est tel que défini en a),
ou un halogénure d'alkyle ayant jusqu'à 6 atomes de carbone, pour aboutir à une xanthine convenablement substitué en 3,7 et on la fait ensuite réagir avec un composé de formule II ou de formule III, et on élimine ensuite le groupe protecteur.

7. Médicaments pour le traitement de maladies qui peuvent apparaître après des traumatismes crânio-cérébraux, caractérisés par une teneur efficace en au moins un dérivé de xanthine de formule I selon les revendications 4 et 5.

8. Procédé pour la fabrication d'un médicament selon une ou plusieurs des revendications 4 et 5, destiné au traitement de maladies qui peuvent apparaître après des traumatismes crânio-cérébraux, caractérisé en ce que l'on met sous une forme d'administration appropriée au moins un dérivé de xanthine de formule I et/ou au moins un de ses sels physiologiquement acceptables avec un véhicule physiologiquement acceptable et d'autres substances actives, additifs ou adjuvants appropriés.

## Revendications (Revendications pour l'(les) Etat(s) contractant(s) suivant(s): ES, GR)

1. Utilisation d'au moins un dérivé de xanthine de formule I, 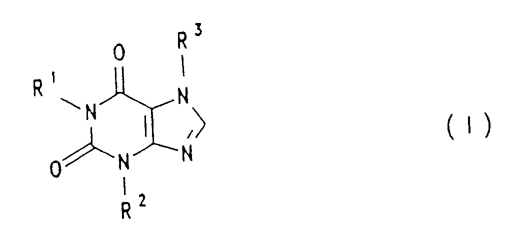 et/ou
d'au moins un de ses sels physiologiquement acceptables, formule dans laquelle
R¹ représente
a) un groupe oxoalkyle ayant de 3 à 8 atomes de carbone, dont la chaîne carbonée peut être linéaire ou ramifiée,
b) un groupe hydroxyalkyle ayant de 1 à 8 atomes de carbone, dont la chaîne carbonée peut être linéaire ou ramifiée et dont le radical hydroxy représente une fonction alcool primaire, secondaire ou tertiaire, ou
c) un groupe alkyle ayant de 1 à 6 atomes de carbone, dont la chaîne carbonée peut être linéaire ou ramifiée,
R² représente
a) un atome d'hydrogène ou
b) un groupe alkyle ayant de 1 à 4 atomes de carbone, dont la chaîne carbonée peut être linéaire ou ramifiée,
R³ représente
a) un atome d'hydrogène,
b) un groupe alkyle ayant de 1 à 6 atomes de carbone, dont la chaîne carbonée peut être linéaire ou ramifiée,
c) un groupe alkyle ayant de 1 à 6 atomes de carbone, dont la chaîne carbonée est interrompue par un atome d'oxygène, ou
d) un groupe oxoalkyle ayant de 3 à 8 atomes de carbone, dont la chaîne carbonée peut être linéaire ou ramifiée,
pour la fabrication de médicaments destinés au traitement de lésions diffuses du tissu cérébral qui sont en relation avec l'activation de la microglie et qui peuvent apparaître après des traumatismes crânio-cérébraux.

2. Utilisation selon la revendication 1, caractérisée en ce que l'on utilise au moins un dérivé de xanthine de formule I dans lequel
R¹ représente
a) un groupe oxoalkyle ayant de 4 à 6 atomes de carbone, dont la chaîne carbonée est linéaire, ou
b) un groupe alkyle ayant de 3 à 6 atomes de carbone,
R² représente un groupe alkyle ayant de 1 à 4 atomes de carbone,
R³ représente
a) un groupe alkyle ayant de 1 à 4 atomes de carbone, ou
b) un groupe oxoalkyle ayant de 3 à 6 atomes de carbone, et/ou au moins un de ses sels physiologiquement acceptables.

3. Utilisation de la 1-(5-oxohexyl)-3-méthyl-7-n-propylxanthine ou d'au moins un de ses sels physiologiquement acceptables, pour la fabrication de médicaments destinés au traitement de lésions diffuses du tissu cérébral qui sont en relation avec l'activation de la microglie et qui peuvent apparaître après des traumatismes crânio-cérébraux.

4. Procédé pour la préparation des dérivés de xanthine de formule I 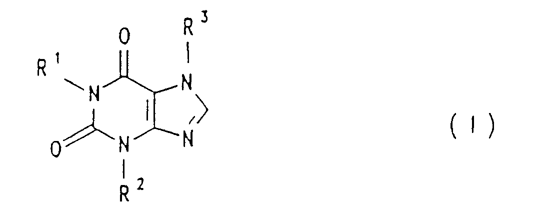 dans laquelle
R¹ représente
a) un groupe oxoalkyle ayant de 3 à 8 atomes de carbone, dont la chaîne carbonée peut être linéaire ou ramifiée, ou
b) un groupe hydroxyalkyle ayant de 1 à 8 atomes de carbone, dont la chaîne carbonée peut être linéaire ou ramifiée et dont le radical hydroxy représente une fonction alcool primaire, secondaire ou tertiaire,
R² représente un atome d'hydrogène,
R³ représente
a) un atome d'hydrogène,
b) un groupe alkyle ayant de 1 à 6 atomes de carbone, dont la chaîne carbonée peut être linéaire ou ramifiée,
c) un groupe alkyle ayant de 1 à 6 atomes de carbone, dont la chaîne carbonée est interrompue par un atome d'oxygène, ou
d) un groupe oxoalkyle ayant de 3 à 8 atomes de carbone, dont la chaîne carbonée peut être linéaire ou ramifiée,
caractérisé en ce que
a) on fait réagir des xanthines protégées sur R² et R³, avec un composé de formule II ou de formule III 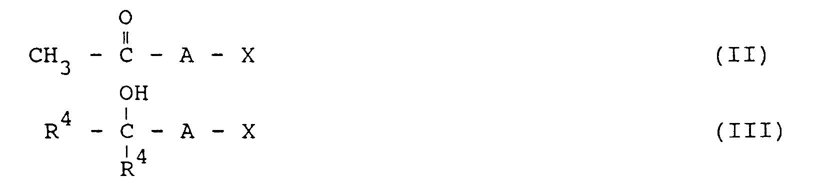 dans des conditions basiques, A représentant un groupe alkyle ayant de 1 à 6 atomes de carbone, X représentant un atome d'halogène, tel qu'un atome de fluor, chlore, brome ou iode, et R⁴ représentant un atome d'hydrogène et/ou le groupe méthyle, et ensuite on élimine le groupe protecteur,
b) on fait réagir des xanthines protégées sur R², avec un composé de formule II ou de formule IV
CH₃-CₙH₂-O-CₘH₂-X
dans laquelle
n représente un nombre entier allant de 0 à 4 et m représente un nombre entier allant de 1 à 5, étant entendu que la somme de n et m n'excède pas 5 et X est tel que défini en a),
ou un halogénure d'alkyle ayant jusqu'à 6 atomes de carbone, pour aboutir à une xanthine convenablement substitué en 3,7 et on la fait ensuite réagir avec un composé de formule II ou de formule III, et on élimine ensuite le groupe protecteur.

5. Procédé selon la revendication 4, caractérisé en ce que l'on prépare un dérivé de xanthine de formule I dans lequel
R¹ représente un groupe hydroxyalkyle ayant de 1 à 6 atomes de carbone, dont la chaîne carbonée est linéaire ou ramifiée, et dont le radical hydroxy représente une fonction alcool primaire, secondaire ou tertiaire,
R² représente un atome d'hydrogène,
R³ représente
a) un atome d'hydrogène ou
b) un groupe alkyle ayant de 1 à 6 atomes de carbone, dont la chaîne carbonée est interrompue par un atome d'oxygène.

6. Procédé pour la fabrication d'un médicament destiné au traitement de maladies qui peuvent apparaître après des traumatismes crânio-cérébraux, caractérisé en ce que l'on met sous une forme d'administration appropriée au moins un dérivé de xanthine de formule I et/ou au moins un de ses sels physiologiquement acceptables avec un véhicule physiologiquement acceptable et d'autres substances actives, additifs ou adjuvants appropries.
